# EUROPEAN PATENT APPLICATION

(11) **EP 1 985 602 A1**
(43) Date of publication of application: **29.10.2008**
(21) Application number: 06843233.5
(22) Date of filing: 26.12.2006
(51) Int. Cl.: C07C 37/86, C07B 61/00, C07C 37/20, C07C 37/48, C07C 39/16

(54) **PROCESS FOR PRODUCING BISPHENOL A**

(30) Priority: 14.02.2006 JP 2006036829
(71) Applicant: Idemitsu Kosan Co., Ltd., Chiyoda-ku Tokyo 100-8321 (JP)
(72) Inventor: YOSHITOMI, Kazuyuki, Ichihara-shi, Chiba 299-0193 (JP); KODAMA, Masahiro, Ichihara-shi, Chiba 299-0193 (JP); MASUDA, Shuichi, Ichihara-shi, Chiba 299-0193 (JP)
(74) Representative: Vossius & Partner
(86) International application number: PCT/JP2006/325849
(87) International publication number: WO 2007/094124

(57) **Abstract**

Upon isomerizing bis(hydroxyphenyl) propanes other than bisphenol A (2,2-bis(4-hydroxyphenyl) propane) into bisphenol A by contacting a liquid containing bis(hydroxyphenyl) propanes to a strongly acidic cation-exchange resin, the water concentration of the liquid containing bis(hydroxyphenyl) propanes is regulated at 0.2 to 0.9% by mass. Thereby, the operating life of the isomerization catalyst used in the method for producing bisphenol A including an isomerization step can be extended, and bisphenol A can be produced efficiently. In addition, the amount of the catalyst disposed can be reduced.

## Description

### [Technical Field]

The present invention relates to a method for producing bisphenol A (2,2-bis(4-hydroxyphenyl) propane) including an isomerization step, specifically to a method for producing bisphenol A efficiently by extending the operating life of an isomerization catalyst.

### [Background Art]

Bisphenol A is an essential compound as a source material for epoxy resin or polycarbonate resin. The use and demand of bisphenol A have been growing in recent years.
Bisphenol A is usually obtained by: condensating acetone and an excess amount of phenol in the presence of an acidic catalyst; cooling the concentrated liquid of the resulting reaction mixture so as to crystallize an adduct of bisphenol A and phenol and to separate the concentrated liquid into the adduct and a mother liquid; and decomposing the adduct.

The mother liquid obtained upon crystallizing the adduct of bisphenol A and phenol contains, besides bisphenol A, a number of components such as 2-(2-hydroxyphenyl)-2-(4-hydroxyphenyl) propane (hereinafter, referred to as o,p'-form) that are isomerizable into a p,p'-form. Therefore, in order to increase the yield of a product bisphenol A, it is important that these isomerizable components are isomerized into the p,p'-form and recovered. In this isomerization, usually a sulfonic acid type cation-exchange resin is used as a catalyst. However, the catalyst degrades rapidly and requires frequent catalyst replacement, thereby lowering the operation rate of a bisphenol A production plant.

Examples of known methods for isomerizing the mother liquid that is obtained after the adduct or bisphenol A and phenol is crystallized and separated from the condensation reaction mixture of phenol and acetone include: a method in which a sulfonic acid type cation-exchange resin washed with phenols is used (Patent Document 1); a method in which the reaction is performed in the presence of water in the early stage thereof (Patent Document 2); a method in which a part of the mother liquid is isomerized, and then circulated back to the condensation reaction step or the concentration step (Patent Document 3); and a method in which after all of the mother liquid is isomerized followed by a part of the resulting isomerized liquid is circulated back to the condensation reaction step, the concentration step, or the crystallization and solid/liquid separation step while bisphenol A or phenol is recovered from the remaining isomerized liquid (Patent Document 4); and others.
However, presently no document, which describes about extending the operating life of a sulfonic acid type cation-exchange resin that is used as a catalyst, is found.

Patent Document 1: Japanese Patent Laid-Open Publication No. Sho.62-201833,
Patent Document 2: Japanese Patent Laid-Open Publication No. Hei.5-339187,
Patent Document 3: Japanese Patent Laid-Open Publication No. Hei.8-333290,
Patent Document 4: Japanese Patent Laid-Open Publication No. 2004-359594.

### [Disclosure of the Invention]

### [Problems to be Solved by the Invention]

Under the circumstances described above, it is an objective of the present invention to provide a method for producing bisphenol A efficiently by extending the operating life of an isomerization catalyst used in a method for producing bisphenol A including an isomerization step.

### [Means for Solving the Problems]

The present inventors have made intensive studies to address the problems and found that the operating life of the isomerization catalyst can be extended by regulating the water concentration of a liquid that contains bis(hydroxyphenyl) propanes and is contacted to the isomerization catalyst, and that a commercial production plant is allowed to operate continuously over two to three years and bisphenol can be produced efficiently. The present invention is based on this finding.

Namely, the present invention provides the following method for producing bisphenol A.
1. A method for producing bisphenol A characterized in that the water concentration of a liquid containing bis(hydroxyphenyl) propanes is regulated at 0.2 to 0.9% by mass upon isomerizing bis(hydroxyphenyl) propanes other than bisphenol A (2,2-bis(4-hydroxyphenyl) propane) into bisphenol A by contacting the liquid containing bis(hydroxyphenyl) propanes to a strongly acidic cation-exchange resin.
2. The method for producing bisphenol A as described in (1), including the steps: (A) a condensation reaction step of reacting acetone and an excess amount of phenol in the presence of an acidic catalyst; (B) a concentration step of concentrating the reaction mixture obtained in the condensation reaction step; (C) a crystallization and solid/liquid separation step of crystallizing an adduct of bisphenol A and phenol by cooling the concentrated liquid obtained in the concentration step and separating the concentrated liquid into the adduct and a mother liquid; (D) an isomerization step of isomerizing bis(hydroxyphenyl) propanes other than bisphenol A into bisphenol A by contacting all of the mother liquid obtained in the crystallization and solid/liquid separation step to a strongly acidic cation-exchange resin; (E) a recovering step of circulating a part of the isomerized liquid back to at least one step selected from (A) the condensation reaction step, (B) the concentration step, and (C) the crystallization and solid/liquid separation step while recovering bisphenol A and phenol from a part of the isomerized liquid; (F) an adduct decomposition step of removing phenol from the adduct of bisphenol A and phenol and recovering bisphenol A; and (G) a prilling step of granulating the bisphenol A obtained in the adduct decomposition step, wherein the water concentration of the mother liquid is regulated at 0.2 to 0.9% by mass in (D) the isomerization step.
3. The method for producing bisphenol A as described in (2), wherein cooling is performed by vacuum-vaporization crystallization accompanied by water addition in (C) the crystallization and solid/liquid separation step.

### [Effect of the Invention]

In the method for producing bisphenol A according to the present invention, by regulating the water concentration in a mother liquid that is obtained after the adduct of bisphenol A and phenol is crystallized and separated from a condensation reaction mixture of phenol and acetone and is supplied to an isomerization catalyst, the operating life of the isomerization catalyst can be extended and a commercial production plant is allowed to operate continuously over two to three years, thereby bisphenol is produced efficiently. In addition, the amount of the catalyst disposed is reduced, so that can be brought about an environmental effect such as the reduction of a large amount of phenol waste water produced in the course of disposing safely the catalyst.

### [Best Mode for Carrying Out the Invention]

The method for producing bisphenol A according to the present invention is characterized in that, upon isomerizing bis(hydroxyphenyl) propanes other than bisphenol A into bisphenol A by contacting a liquid containing bis(hydroxyphenyl) propanes to a strongly acidic cation-exchange resin, the water concentration of the liquid containing bis(hydroxyphenyl) propanes is regulated at 0.2 to 0.9% by mass. Specifically, bisphenol A is produced through the following steps: (A) a condensation reaction step; (B) a concentration step; (C) a crystallization and solid/liquid separation step; (D) an isomerization step; (E) a recovering step of an isomerized liquid; (F) an adduct decomposition step; and (G) a prilling step. Hereinafter, each step is explained in detail.

### (A) Condensation reaction step

In the condensation reaction step, phenol and acetone are reacted in the presence of an acidic catalyst. Source phenol and acetone are reacted in a manner that phenol is in excess stoichiometrically. The molar ratio of phenol/acetone is usually in the range of from 3 to 30 and preferably from 5 to 20. The reaction temperature is usually 50 to 100°C. The reaction pressure is usually normal pressure to 1.5 MPa and preferably normal pressure to 0.6 MPa. The catalyst includes usually a strongly acidic cation-exchange resin such as a sulfonic acid type. May be included a catalyst that is given by neutralizing a part of the strongly acidic cation-exchange resin with an auxiliary catalyst such as mercapto alkylamine. For example, may be included a catalyst whose sulfonic acid group is neutralized by about 5 to about 30 mol% with 2-mercapto ethylamine, 3-mercapto propylamine, N,N-dimethyl-3-mercapto propylamine, N,N-di-n-butyl-4-mercapto butylamine, 2,2-dimethyl thiazolidine, and the like. Condensation reaction between phenol and acetone is carried out in a fixed-bed flow process that is a continuous plug-flow process or in a suspension-bed batch process. In the case of the fixed-bed flow process, the liquid hourly space velocity (LHSV) of a source liquid introduced into a reactor is about 0.2 to about 50 hr⁻¹. In the case of the suspension-bed batch-wise process, the amount of a resin catalyst is in the range of generally from 20% to 100% by mass with respect to the source liquid and the reaction time is about 0.5 to about 5 hours, although they depend on the reaction temperature and pressure.

### (B) Concentration step

In the concentration step, the reaction mixture obtained in the condensation reaction step is concentrated. The reaction mixture from the condensation reaction step is usually concentrated in two-steps. In a first concentration step, unreacted acetone, reaction product water, and others are removed by vacuum distillation or the like. Vacuum distillation is carried out usually at a temperature of 30 to 180°C under a pressure of 13 to 67 kPa. Then, in a second concentration step, phenol is distilled out so as to control the bisphenol A concentration. The bisphenol A concentration is regulated at preferably 20 to 60% by mass at this time. At a bisphenol A concentration of 20% by mass or more, yield becomes increased. On the other hand, at a bisphenol A concentration of 60% by mass or less, solidification temperature is lowered, thereby enabling easy transportation. Therefore, usually in the first concentration step, the reaction mixture is concentrated in advance and adjusted at a concentration within the above range. Usually it is desirable to carry out the second concentration under a pressure of 4 to 40 kPa and at a temperature of 70 to 140°C.

### (C) Crystallization and solid/liquid separation step

In the crystallization and solid/liquid separation step, the concentrated liquid obtained in the concentration step is cooled so as to crystallize an adduct of bisphenol A and phenol and to separate the concentrated liquid into the adduct and a mother liquid. The concentrated liquid from the concentration step is usually cooled from a temperature of 70 to 140°C to a temperature of 35 to 60°C, so that an adduct of bisphenol A and phenol is crystallized, and that the concentrated liquid is changed into a slurry liquid. The concentrated liquid is cooled by an external heat-exchanger or by way of vacuum-vaporization crystallization in which heat is removed by vaporization of water that is added to a crystallizer.
After that, the slurry liquid is subjected to solid/liquid separation. The mother liquid obtained in this crystallization and solid/liquid separation step contains reaction water, so that it is usually introduced into a dehydration tower. Note that, a part of the water-containing mother solution may be circulated back to the crystallizer. The composition of the mother liquid obtained after dehydration is usually as: phenol is 65 to 85 % by mass; bisphenol A is 10 to 20% by mass; and by-products such as 2,4'-isomers are 5 to 15% by mass. The mother liquid contains a large amount of impurities such as 2,4'-isomers. This mother liquid is treated in the following isomerization step.

The adduct recovered by the solid/liquid separation is forwarded to the adduct decomposition step (F) in which phenol is removed to obtain high-purity bisphenol A.
A solid that contains mainly the adduct and is deposited on the surface of a filter of the solid/liquid separator after filtration is subjected to washing with a cleaning liquid. The cleaning liquid may include phenol that is recovered by evaporation, source phenol, water, and a water-phenol mixed liquid, and also may include a solution similar to a bisphenol A saturated phenol solution. The much the amount of the cleaning liquid used the better of course, from the viewpoint of cleaning efficiency. However, the amount has an own upper limit considering the dissolution loss of crystals and the circulation, recovery and reuse of the cleaning liquid. The amount considered to be most efficient is usually about 0.1 to about 10 times of the crystals on a mass basis.
Note that, the crystals may be re-dissolved after the crystallization and solid/liquid separation, and the crystallization and solid/liquid separation may be repeated. The impurities incorporated in the adduct crystals are decreased successively by repeating the crystallization and solid/liquid separation in multi-stages.
As a cleaning liquid used in this occasion to wash the solution obtained by redissolution and the solid that is obtained by solid/liquid separation and contains mainly the adduct, phenol that is recovered by evaporation, source phenol, water, and a water-phenol mixed liquid, and also a solution similar to a bisphenol A saturated phenol solution may be used in each stage. The mother liquid obtained by re-crystallization and solid/liquid separation may be recycled to the crystallization step in the former stage.
The solid/liquid separator used for the solid/liquid separation is not particularly limited as long as the separator is the one that is usually used, but examples of the separator may include a belt filter, a drum filter, a tray filter, a centrifugal separator, and the like.

### (D) Isomerization step

In the isomerization step, the mother liquid obtained in the crystallization and solid/liquid separation step is contacted to a strongly acidic cation-exchange resin so as to isomerize bis(hydroxyphenyl) propanes other than bisphenol A into bisphenol A.
Note that, as a method for the isomerization, there may be mentioned: a method (D1) as described in Patent Document 4 in which all of the mother liquid obtained in the crystallization and solid/liquid separation step is treated in the isomerization step, and then a part of the resulting isomerized liquid is circulated back to at least one step selected from (A) the reaction step, (B) the concentration step, and (C) the crystallization and solid/liquid separation step, and bisphenol A or phenol is recovered from the remaining isomerized liquid; and a method (D2) as described in Patent Document 3 in which a part of the mother liquid obtained in the crystallization and solid/liquid separation step is treated in the isomerization step and circulated back to (A) the reaction step or (C) the crystallization and solid/liquid separation step, and phenol is recovered from the remaining mother liquid. In the present invention, may be applied either method (D1) or (D2), but (D1) is advantageous because the isomerization is proceeded efficiently when all of the mother liquid is treated in the isomerization step and a large amount of bisphenol A can be recovered.

Examples of bis(hydroxyphenyl) propanes other than bisphenol A that are contained in the mother liquid obtained in the crystallization and solid/liquid separation step include an o,p'-form of bis(hydroxyphenyl) propane, and further include 2-(2-hydroxyphenyl)-2-(3-hydroxyphenyl) propane (o,m'-form); 2-(2-hydroxyphenyl)-2-(2-hydroxyphenyl) propane (o,o'-form); 2-(3-hydroxyphenyl)-2-(3-hydroxyphenyl) propane (m,m'-form); 2-(3-hydroxyphenyl)-2-(4-hydroxyphenyl) propane (m,p'-form); trisphenols; and the like.

In the present invention, as the mother liquid containing bis(hydroxyphenyl) propanes that is contacted to a strongly acidic cation-exchange resin, may be used the mother liquid as it is that is obtained after the adduct of bisphenol A and phenol is crystallized and separated from the reaction mixture obtained by condensating phenol and acetone, but may be used a liquid that is given by adding to the mother liquid a phenol cleaning liquid obtained after the adduct crystallized and separated is washed, or may be used the mother liquid whose concentration was regulated by adding or distilling out phenol.

The sulfonic acid type cation-exchange resin used for the isomerization in the present invention is not particularly limited as long as the resin is a strongly acidic cation-exchange resin having a sulfonic acid group. Examples of the resin include sulfonated styrene/divinylbenzene copolymer, sulfonated cross-linked styrene polymer, phenol formaldehyde-sulfonic acid resin, benzene formaldehyde-sulfonic acid resin, and the like. These may be used one kind solely or in a combination of two or more kinds.
Further, as the isomerization catalyst, may be sometimes used a catalyst that is given by neutralizing a part of a strongly acidic cation-exchange resin with an auxiliary catalyst such as mercapto alkylamine. Examples of the catalyst may include the one whose sulfonic acid groups are neutralized by 5 to 30 mol% with 2-mercapto ethylamine, 3-mercapto propylamine, N,N-dimethyl-3-mercapto propylamine, N,N-di-n-butyl-4-mercapto butylamine, 2,2-dimethylthiazolidine, or the like.

As described above, the concentrated liquid obtained in the concentration step is cooled by an external heat exchanger or by way of vacuum-vaporization crystallization in which heat is removed by vaporization of water that is added to a crystallizer . The vacuum-vaporization crystallization accompanied by water addition is suitably applied because the vaporization heat is used effectively.
When the crystallization is operated by the vacuum-cooling crystallization accompanied by water addition, water is remained in an amount of 3 to 8% by mass in the mother liquid. Hence, usually the mother liquid is subjected to dehydration.
Examples of a preferred dehydration method of regulating the water concentration in the mother liquid may include a method using an evaporator under a reduced pressure or using a distillation tower (hereinafter, referred to as dehydration tower) loaded with a filler.
In the dehydration tower, water and phenol are distilled out from the tower head and a mother liquid having a regulated water concentration is drawn out from the tower bottom. The water concentration of the mother liquid at the tower bottom is regulated by controlling the temperature of the tower bottom with a reboiler heat medium.

Note that, in the case of repeating two or more times the operation of crystallization and solid/liquid separation, the amount of the impurities such as 2,4-isomers remained in the mother liquid after the second time of the operation becomes low, so that the mother liquid after the second time is not introduced into an isomerization reactor but is preferably circulated back to at least one step selected from (A) the condensation reaction step, (B) the concentration step, and (C) the crystallization step of the former stage (such as the crystallization tank of the first time). The water concentration of the mother liquid when it is circulated back to the condensation reaction step is preferably from 0.01 to 0.1% by mass, considering the activity of the reaction catalyst. When this operation of crystallization is performed by the vacuum-vaporization crystallization, the above water concentration can be attained by applying the same dehydration as described above.
The mother liquid forwarded from the crystallization and solid/liquid separation step to the dehydration step may be introduced into the dehydration tower after the liquid is heated in advance with a preliminary heating apparatus. In addition, the mother liquids forwarded from the plural stages of the vacuum-vaporization crystallization accompanied by water addition, each is preferably treated with each dehydration tower separated from each other under the same reduced pressure condition. By selecting the same reduced pressure condition, phenol and water discharged out of the head of each tower can be condensed with one condenser while the tower bottom temperature of each tower is regulated with each reboiler heat medium. Thereby, the condensed water can be reused for water addition in the vacuum-vaporization crystallization.

Isomerization is preferably performed by introducing the mother liquid in a downflow direction into a fixed-bed reactor (isomerization reactor) loaded with a strongly acidic cation-exchange resin. The strongly acid cation-exchange resin may include a sulfonic acid type cation-exchange resin as mentioned above. The isomerization temperature is in the range of preferably from 60 to 100°C and more preferably from 70 to 80°C. At a temperature of 60°C or more, bisphenol A solidification in the mother liquid can be avoided. At a temperature of 100°C or less, can be avoided a problem of elimination of sulfonic acid from the catalyst or degradation of product grade due to bisphenol A decomposition by acid.
In the case of the fixed-bed isomerization reactor, the liquid hourly space velocity (LHSV) is usually preferably 0.1 to 10 hr⁻¹ and more preferably 0.2 to 1.0 hr⁻¹. At an LHSV of 0.1 hr⁻¹ or more, the amount of by-products is reduced. At 10 hr⁻¹ or less, a higher conversion can be attained.

In the present invention, the water concentration of the mother liquid introduced into the isomerization reactor is 0.2 to 0.9% by mass and preferably 0.3 to 0.5% by mass. At 0.9% by mass or less, the isomerization catalyst is allowed to keep a high activity. When the water concentration is 0.2% by mass or more, the ion exchange resin serving as a catalyst is swollen with water, so that the micropores of the resin become large and that the micropores are not easily clogged with heavy impurities. Due to this, the degradation speed of the catalyst is lowered and the operating life of the catalyst is extended, thereby decreasing the frequency of catalyst replacement.
In a commercial plant, when the catalyst of the isomerization reactor starts to degrade, impurities are concentrated, thereby the product grade lowers. In order to secure the product grade, the operating rate is required to be cut if the operation continues without interruption, or the operation is required to be interrupted for catalyst replacement. Therefore, extended operating life of the catalyst in the isomerization reactor provides an effect of stable operation without cutting the operating rate or an advantage of allowing the catalyst replacing timing to coincide with the timing of halting or full-checking of the production plant. In addition, the following environmental effect is brought about: the amount of the catalyst disposed (usually, 30 to 100 m³ in a commercial plant) is reduced, so that can be reduced the amount of phenol waste water (usually, 300 to 1,000 m³) that is produced when the phenol incorporated in the catalyst is washed out with a larger amount of water in order to dispose the catalyst safely.
The degradation of the catalyst in the isomerization reactor can be evaluated in terms of the following conversion of 2,4-isomers. In a commercial plant, about 30% of this conversion of 2,4-isomers is desirably used as an economical guide for catalyst replacement.
Here, the conversion of 2,4-isomers is calculated as: {(2,4-isomer concentration at the reactor inlet) - (2,4-isomer concentration at the reactor outlet)} / (2,4-isomer concentration at the reactor inlet).
The water concentration of the mother liquid introduced into the isomerization reactor may be regulated with the operating temperature of the dehydration tower or by adding water.

### (E) Recovering step for isomerized liquid

The isomerized liquid is circulated back to at least one step selected from (A) the condensation reaction step, (B) the concentration step, and (C) the crystallization and solid/liquid separation step so as to recover bisphenol A.
In a flow sequence in accordance with the method (D1), all of the mother liquid obtained in the crystallization and solid/liquid separation step is treated in the isomerization step; a part of the resulting isomerized liquid is circulated back to at least one step selected from (A) the condensation reaction step, (B) the concentration step, and (C) the crystallization and solid/liquid separation step; the remaining resulting isomerized liquid is blown so as to avoid impurity accumulation. In this way, bisphenol A or phenol is recovered from the isomerized liquid.
The blow liquid forwarded from the isomerization step contains bisphenol A in an amount of about 15 to about 20% by mass, impurities such as 2,4'-isomers in an amount of about 5 to about 10% by mass, and the remainder of phenol.
This blow liquid is concentrated and cooled so as to crystallize an adduct of bisphenol A and phenol. After solid/liquid separation, the adduct is fused and circulated back to the concentration step and/or the crystallization and solid/liquid separation step. The mother liquid obtained after the solid/liquid separation is disposed as tar after phenol is recovered from the mother liquid.
The blown isomerized liquid is concentrated by distillation usually under a pressure of about 3 to about 10 kPa and at a temperature of about 90 to about 130°C so as to recover phenol.
The resulting concentrated liquid is cooled so as to crystallize an adduct of bisphenol A and phenol. After the adduct is subjected to solid/liquid separation, it is circulated back to the concentration step and others. Phenol is recovered from the mother liquid obtained after the solid/liquid separation also by distillation in almost the same manner as above. The recovered phenol is reused as a cleaning liquid in the solid/liquid separation step or as a source material in the condensation reaction step.

### (F) Adduct decomposition step

In the adduct decomposition step, phenol is removed from the adduct of bisphenol A and phenol so as to recover bisphenol A. In the adduct decomposition step phenol is removed from the adduct recovered by solid-liquid separation so as to obtain high-purity bisphenol A.
Namely, the adduct is heated and fused at a temperature of about 100 to about 160°C to decompose it into bisphenol A and phenol. Most of the phenol is removed from the resulting melt with a distillation drum or the like, and the remaining phenol is further removed by steam stripping, so that a melt of bisphenol A is obtained.

### (G) Prilling step

In the prilling step, the melt of bisphenol A obtained in the adduct decomposition step is prilled so as to separate a product bisphenol A. In the case of spray granulation, the melt of the bisphenol A obtained in the adduct decomposition step is usually forwarded to the head of the prilling tower and sprayed through a number of holes opened in a nozzle plate placed at the head of the tower. The melt thus sprayed is cooled with a circulating gas flowing upward from the bottom of the prilling tower. The resulting granular solids called as prills are taken out from the bottom to obtain product bisphenol A prills.
Further, in an another prilling method, the melt of bisphenol A is supplied to a cooled rotating drum to obtain a flaky product of bisphenol A.

### Example

Hereinafter, the present invention will be described in detail with reference to the following examples, but it should be construed that the present invention is in no way limited to those examples.
Note that, in the following examples, the conversion of 2,4-isomers is calculated as: {(2,4-isomer concentration at the reactor inlet) - (2,4-isomer concentration at the reactor outlet)} / (2,4-isomer concentration at the reactor inlet).
Further, the time when the conversion of 2,4-isomers reaches 30% is selected as the replacement timing of the isomerization catalyst. The ratio of the replacement timing with respect to the replacement timing (catalyst operating life) for the isomerization catalyst of comparative example 1 where the mother liquid contacting to the isomerization catalyst contains water in very little amount is defined as a catalyst operating life ratio.

### Example 1

Through a fixed-bed reactor tower loaded with a cation-exchange resin ("DIAION SK104H" (trade name), manufactured by Mitsubishi Chemical Corporation) whose sulfonic acid groups were neutralized partly by 20 mol% with 2-mercapto ethylamine, phenol and acetone in a molar ratio of 10:1 were passed continuously at an LHSV of 3 hr⁻¹. They were reacted at 75°C.
After acetone, water, and others were removed from the resulted reaction mixture by vacuum distillation at a tower bottom temperature of 170°C and under a pressure of 67 kPa, phenol was further removed by vacuum-distillation at a tower bottom temperature of 130°C and under a pressure of 14 kPa so as to concentrate bisphenol A to 40% by mass and to obtain a phenol/bisphenol A solution.
Then, water was added to the phenol/bisphenol A solution having a bisphenol A concentration of 40% by mass. The solution was kept cooled at 50°C under vacuum so as to crystallize an adduct of bisphenol A and phenol. In this way, a slurry liquid was obtained.

Isomerization was carried out by using as an isomerization reactor a fixed-bed (1.3 cm of diameter, 64 cm of height) loaded with a sulfonic acid type cation-exchange resin ("DIAION SK-104H" (trade name), manufactured by Mitsubishi Chemical Corporation) preliminary swollen with phenol, and as a source liquid, a mother liquid that was obtained by filtering off the crystallized adduct of bisphenol A and phenol from the slurry liquid obtained as described above.
The water content was regulated by distilling the mother liquid under a reduced pressure of 30 kPa. The composition of the mother liquid was as follows: bisphenol A was 11.0% by mass; 2,4-isomers was 4.0% by mass; the other impurities were 3.0% by mass; water was 0.40% by mass; and the remainder of phenol. The mother liquid was passed through the isomerization reactor under normal pressure, at a temperature of 75°C, and at an LHSV of 1 hr⁻¹. The reaction product was analyzed with time.
The result of the 2,4-isomer conversion measured with respect to the reaction time is shown in Table 1. At a reaction time of 8,000 hours, the 2,4-isomer conversion was 30% and the catalyst operating life ratio was 2.29. From this result, in a commercial plant with an LHSV of 0.3 hr⁻¹, the time when the 2,4-isomer conversion becomes 30% was estimated to be 24,000 hours, which allowed a continuous operation over 3 years.

### Example 2

Except that the water content of the mother liquid obtained after the filtration of the adduct of bisphenol A and phenol in Example 1 was regulated at 0.80% by mass, similarly to Example 1, the 2,4-isomer conversion was measured with respect to the reaction time. The result is shown in Table 1. At a reaction time of 8,000 hours, the 2,4-isomer conversion was 30% and the catalyst operating life ratio was 2.29. From this result, in a commercial plant with an LHSV of 0.3 hr⁻¹, the time when the 2,4-isomer conversion becomes 30% was estimated to be 24,000 hours, which allowed a continuous operation over 3 years.

### Comparative Example 1

Except that the water content of the mother liquid obtained after the filtration of the adduct of bisphenol A and phenol in Example 1 was regulated at 0.03% by mass, similarly to Example 1, the 2,4-isomer conversion was measured at every 500 hours of reaction time. The result is shown in Table 1. At a reaction time of 3,500 hours, the 2,4-isomer conversion reached 30% and the isomerization catalyst replacement time (catalyst operating life) was arrived. From this result, in a commercial plant with an LHSV of 0.3 hr⁻¹, the time when the 2,4-isomer conversion becomes 30% was estimated to be 9,000 hours, which did not allow a continuous operation for 2 years or more.

### Comparative Example 2

Except that the water content of the mother liquid obtained after the filtration of the adduct of bisphenol A and phenol in Example 1 was regulated at 1.50% by mass, similarly to Example 1, the 2,4-isomer conversion was measured with respect to the reaction time. The result is shown in Table 1. At a reaction time of 200 hours, the 2,4-isomer conversion reached 30% and the isomerization catalyst replacement time (catalyst operating life) was arrived. From this result, in a commercial plant with an LHSV of 0.3 hr⁻¹, the time when the 2,4-isomer conversion becomes 30% was estimated to be 500 hours, which was not adapted to the operating condition of a commercial plant.

**Table 1**

| | | Example 1 | Example 2 | Comparative Example 1 | Comparative Example 2 |
|---|---|---|---|---|---|
| Water concentration (% by mass) | | 0.40 | 0.80 | 0.03 | 1.50 |
| 2,4-Isomer conversion (%) | | | | | |
| Reaction time | 200hr | 45 | 43 | 46 | 30 |
| | 500hr | 42 | 36 | 45 | 29 |
| | 1000hr | 39 | 38 | 43 | 28 |
| | 2000hr | 39 | 35 | 38 | 28 |
| | 3000hr | 34 | 33 | 33 | 27 |
| | 4000hr | 32 | 32 | 27 | 26 |
| | 8000hr | 30 | 30 | 9 | 24 |
| Catalyst operating life ratio | | 2.29 | 2.29 | 1.00 | 0.06 |

### [Industrial Applicability]

In the method for producing bisphenol A according to the present invention, by controlling the water concentration of a mother liquid supplied to an isomerization catalyst after an adduct of bisphenol A and phenol is crystallized and separated from a reaction mixture of phenol and acetone, the operating life of the isomerization catalyst can be extended and bisphenol can be produced efficiently. In addition, the amount of the catalyst disposed can be reduced.

## Claims

1. A method for producing bisphenol A, comprising:
regulating the water concentration of a liquid containing bis(hydroxyphenyl) propanes at 0.2 to 0.9% by mass upon isomerizing bis(hydroxyphenyl) propanes other than bisphenol A (2,2-bis(4-hydroxyphenyl) propane) into bisphenol A by contacting the liquid containing bis(hydroxyphenyl) propanes to a strongly acidic cation-exchange resin.

2. The method for producing bisphenol A according to claim 1, comprising:
(A) a condensation reaction step of reacting acetone and an excess amount of phenol in the presence of an acidic catalyst; (B) a concentration step of concentrating the reaction mixture obtained in the condensation reaction step; (C) a crystallization and solid/liquid separation step of crystallizing an adduct of bisphenol A and phenol by cooling the concentrated liquid obtained in the concentration step and separating the concentrated liquid into the adduct and a mother liquid; (D) an isomerization step of isomerizing bis(hydroxyphenyl) propanes other than bisphenol A into bisphenol A by contacting all of the mother liquid obtained in the crystallization and solid/liquid separation step to an strongly acidic cation-exchange resin; (E) a recovering step of circulating a part of the isomerized liquid back to at least one step selected from (A) the condensation reaction step,
(B) the concentration step, and (C) the crystallization and solid/liquid separation step while recovering bisphenol A and phenol from a part of the isomerized liquid; (F) an adduct decomposition step of removing phenol from the adduct of bisphenol A and phenol and recovering bisphenol A; and (G) a prilling step of granulating the bisphenol A obtained in the adduct decomposition step, wherein
the water concentration of the mother liquid is regulated at 0.2 to 0.9% by mass in (D) the isomerization step.

3. The method for producing bisphenol A according to claim 2, wherein cooling is performed by vacuum-vaporization crystallization accompanied by water addition in (C) the crystallization and solid/liquid separation step.
